# EUROPEAN PATENT SPECIFICATION

(11) **EP 0 527 623 B1**
(45) Date of publication and mention of the grant of the patent: **16.10.1996**
(21) Application number: 92307284.7
(22) Date of filing: 10.08.1992
(51) Int. Cl.: C07C 45/34, C07C 45/35, C07C 29/50, B01J 31/18

(54) **Allylic oxidation of olefins**
Allylische Oxidation von Olefinen
Oxydation allyliques d'oléfines

(30) Priority: 12.08.1991 US 743627
(43) Date of publication of application: 17.02.1993
(73) Proprietor: SUN COMPANY, INC. (R&M), Philadelphia, PA 19103-1699 (US)
(72) Inventor: Lyons, James E., Wallingford, PA 19086 (US); Ellis, Paul E., Jr., Downingtown, PA 19335 (US)
(74) Representative: Lewin, John Harvey

(56) References cited:
- EP-A- 0 296 712
- EP-A- 0 471 561
- WO-A-88/07988
- BULLETIN DE LA SOCIETE CHIMIQUE DE FRANCE vol. 11-12, 1980, PARIS FR pages 16 - 17 P. KRAUSZ ET AL. 'Propriétés oxydantes et réductrices de quelques porphyrines et bis - porphyrines du manganèse.'

## Description

This invention relates to the allylic oxidation of olefins to produce valuable products. An allylic oxidation is one in which carbon-hydrogen bonds adjacent to an olefinic double bond are selectively oxidized.

The allylic oxidation of an olefin is an important reaction practised to produce a variety of valuable commercial oxidation products including acrolein, acrylic acid, methacrolein, methacrylic acid, acrylonitrile and others. These oxidations are typically carried out at high temperatures in the vapor phase and are not completely selective (J. E. Lyons, Surface Organometallic Chemistry: Molecular Approaches to Surface Catalysis, 97-127, Kluwer Academic Publishers (1988)).

The oxidative and reductive properties of certain manganese porphyrins are detailed in Bull. Soc. Chim. Fr., 1980, vol. 11-12 Supplement, p16-17. Such porphyrins non-selectively oxidise cyclohexene to the corresponding ketone, alcohol and epoxide.

We have found that iron haloporphyrin complexes are uniquely active for catalyzing the allylic oxidation of olefins at very mild temperatures with very rapid rates. Reactions with air or oxygen as the oxidant occur exothermically at room temperature using very small catalyst concentrations. This finding is quite startling since in the presence of metalloporphyrin catalysts, single oxygen atom donors such as bleach, alkyl hydroperoxides, and hydrogen peroxide give olefin epoxidation rather than predominant allylic oxidation (W.A. Nugent and J. W. Mayer, Metal-Ligand Multiple Bonds, Wiley, New York, 248-251 (1988)).

WO 88/07988 discloses tetraphenyl porphyrins which are beta-substituted by fluoro or chloro and/or bear electronegative substituents on the phenyl including one or two water solubilizing substituents. The new porphorins are particularly suitable as catalysts in a variety of oxidative reactions and methods. In particular, the single-oxygen atom donor iodosopentafluorobenzene is used to selectively epoxidate cyclohexane in the presence of a β-chlorinated-mesotetraphenylhemin.

EP 0 296 712 discloses the use of iron coordination complexes containing a halogenated ligand for the oxidation of hydrocarbons. In particular, reference is made to the use of such complexes for the selective oxidation of alkanes to the corresponding alcohols and ketones.

The catalyst used according to the invention is a metal coordination complex catalyst containing a transition metal center and a ligand having the structure: 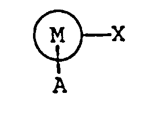 where M is Fe, 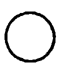 is a porphyrin ligand, X is one or more electron-withdrawing elements or groups, for example chloride, bromide, iodide, fluoride or combinations thereof, or is nitro or cyano, and A is an anion or is absent.

The catalyst used according to one embodiment of the invention is a metal coordination complex catalyst containing an iron center and a halogenated porphyrin ligand, where the ligand is for example a halogenated tetraphenylporphyrin, related porphyrinato ligands, porphycenes or porphenes. Preferably the ligand is a perhalogenated porphyrin. Halogenation of the ligand itself, by replacement of hydrogen atoms therein with halogen atoms, and particularly perhalogenation, increases the activity of these catalysts for the allylic oxidation of olefins according to the invention. The catalyst used in the invention may have, in addition to the halogen atoms in the ligand, a halide anion namely chloride, fluoride, bromide, iodide or combinations thereof. The catalyst may also contain an anion such as azide, hydroxide or nitride. Preferred among the ligands are such macrocyclic groups as halogenated porphyrins. In place of or in addition to halogens, other electron-withdrawing substituents, including nitro and cyano, are useful in enhancing catalyst activity.

The term "ligand" is used herein in its conventional meaning and refers generically to a group or system of atoms which form one or more bonds to a metal ion, i.e. form a coordination complex, and stabilize the coordination complex in desirable oxidation states. Suitable ligands for the present purpose are the well-known porphyrins such as alkyl and aryl porphyrins such as tetraphenylporphyrins, octaethylporphyrins, tetramethylporphyrins and the like. Usually there are 0-12 substituents, alkyl or aryl, on the basic porphyrin structure, the alkyls are C₁-C₄ and the aryls contain 1 or 2 rings which may themselves have alkyl substituents.

The component of the ligand, X, can be fluoride, chloride, bromide, iodide or mixtures thereof, cyano or nitro, but, among the halogens, preferably is one of the first three mentioned, more preferably fluoride. The degree of ligand halogenation should be extensive, i.e. at least 70%, and can be 100%, which is customarily referred to as perhalogenation for which the conventional symbols are F-, Cl-, etc. We have found that in some instances complete halogenation may provide substantially superior results. The degree of nitro or cyano substitution is in the range from 10 t0 100 percent in the catalysts used according to the invention, but in the case of the nitro and cyano substitutions are typically lower than 100 percent because of the difficulty of the nitration and cyanation reactions for preparing the ligand.

The catalysts used can be readily prepared by simple modifications of procedures described in the art for preparing unhalogenated ligands. For example, the unhalogenated Fe(TPP)Cl complex (in which "TPP" is tetraphenylporphyrinato) can be prepared by a standard method in which (TPP)H₂ and Fe(II) chloride are refluxed together in a dimethylformamide solution. Purification is achieved by chromatography. (See, e.g., A. D. Adler et al, J. Inorganic. Nucl. Chem., 32, 2443 (1970)). From these metal salts the corresponding azides may be prepared by metathesis reactions with dissolved NaN₃ or hydrazoic acid.

To prepare the corresponding halogenated ligand, coordination complex of this invention, one or more of the precursors of the ligand are halogenated before the ligand itself is produced by a condensation reaction. Thus synthesis of pentafluorobenzaldehyde followed by condensation with pyrrole yields TPPF₂₀ in which F₂₀refers to twenty fluorine atoms on the four phenyls. Substituting this TPPF₂₀for TPP in the aforementioned method of refluxing in a dimethylformamide solution containing the Fe(II) will yield the corresponding Fe(TPPF₂₀) salt.

By way of specific illustration the perhalogenated metal porphyrin, Fe(TPPF₂₀Br₈ )Cl, iron tetrakispentafluorophenyloctabromoporphyrin chloride is prepared as follows: Under N₂, a flask is charged with 1.0 g of Zn(TPPF₂₀) and 300 ml of CCl₄. This mixture is refluxed with 150 ml of 6M Br₂ for 5 hours and is then allowed to cool to room temperature. After chromatography on basic alumina, 300 mg of pure Zn(TPPF₂₀Br_{B} ) is obtained and characterized by UV/VIS, IR and elemental analysis. The zinc is removed by acid treatment and the iron complex Fe(TPPF₂₀Br₈)Cl, is prepared by FeCl₂ treatment in refluxing DMF. The azide, Fe(TPPF₂₀Br₈)N₃, can be prepared by reaction of the chloride salt with NaN₃ in acetone. The hydroxo salt, Fe(TPPF₂₀Br₈OH), is prepared from the chloro salt by treatment with dilute aqueous KOH in Ch₂Cl₂/H₂O.

The perhalogenated haloporphyrin Fe(TPPF₂₀Cl₈)Cl is prepared as follows: under N₂, 0.5 g of Zn(TPPF₂₀) dissolved in 500 ml of CCl₄ is refluxed for 5 hr. while Cl₂ gas is bubbled slowly through the solution. After cooling the mixture is filtered and chromatographed on alumina, yielding 0.4 g of pure Zn(TPPF₂₀Cl₈). The zinc is removed by trifluoroacetic acid treatment, and the iron is then inserted by reaction with FeCl₂ in DMF. The resulting Fe(TPPF₂₀Cl₈)Cl is characterized by UV/VIS, IR, and elemental analysis. The azide salts are prepared from the chloride salts by methathesis with NaN₃ in acetone. The hydroxo salt, Fe(TPPF₂₀Cl₈ )OH, is prepared from the chloro salt by treatment with dilute aqueous KOH solution in CH₂Cl₂.

The perfluorinated metal porphyrin, iron perfluorotetraphenylporphyrin chloride, Fe(TPPF₂₈)Cl (28 F atoms) can be prepared by the reaction of dilute F₂ gas in N₂ with Zn(TPPF₂₀) in CCl₄, with small added amounts of CoF₃, followed by removal of zinc and incorporation of iron as before. This porphyrin complex is analyzed by IR, UV/VIS, and elemental analysis. The azide salts are prepared from the chloride salts by reaction with NaN₃ in acetone. The hydroxo salt, Fe(TPPF₂₈)OH, is prepared by the dilute aqueous KOH treatment of the chloro salt in CH₂Cl₂.

The preparations of the following iron complexes are examples of tetraalkylporphyrins used in our invention. Freshly distilled pyrrole (0.8g) and trifluoroacetaldehyde methyl hemiacetal (10.9g) are refluxed for 24 hr. in 500 ml of ethanol containing 10 ml of 48% HBr. After neutralization of the mixture and extraction of the crude tetrakis(trifluoromethyl)porphyrin into CH₂Cl₂, the H₂(TTFMP) is purified by chromatography with alumina. Iron is inserted into the H₂ (TTFMP) by FeCl₂ /DMF treatment giving Fe(TTFMP)Cl. The azide and hydroxide complexes are prepared by metathesis with NaN₃ in acetone and aqueous KOH in CH₂Cl₂, respectively. The pyrrolic hydrogens of this porphyrin can be partially or fully halogenated with Br, Cl, or F using the same techniques used for the tetraphenylporphyrins. As an example, dilute F₂ gas treatment of Zn(TTFMP) in the presence of CoF₃ in CCl₄ leads to isolation of the perfluorinated zinc porphyrin, zinc perfluorotetramethylporphyrin Zn(FTMP). Removal of the zinc by strong acid treatment leads to the metal free H₂(FTMP) from which the iron complex Fe(FTMP)Cl can be prepared by FeCl₂ /DMF treatment. The azide, hydroxide, and nitride complexes are prepared in similar fashion to those described before.

When other porphyrin compounds are used similar results are obtained. The excellent catalytic activity of the catalyst depends on the electronic and structural nature of the porphyrin macro structure itself, not on any specific substituted group.

Nitro-substituted metal ligands can be prepared for example by the following procedure: Iron tetrakispentafluorophenylporphine chloride is reacted with nitrogen dioxide (1-8) equivalents in methylene chloride or benzene leading to various amounts of nitration at the beta positions on the ring according to the severity of the reaction conditions. Beta positions left unnitrated are subsequently halogenated using normal chlorination, bromination or fluorination techniques. The general structure for the product is: 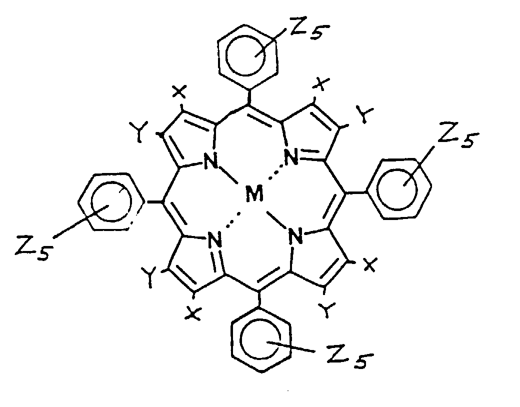 where M is Fe, X is nitro, Y is nitro or Cl or Br or F, and Z is H or Cl or F.

In another preparation, Zn(porphine) is reacted with nitrogen dioxide in methylene chloride to produce Zn(mesotetranitroporphine). The zinc is removed by acid treatment and Fe is inserted by the usual method of ferrous chloride in dimethylformamide.

The beta or pyrrolic hydrogens can be further nitrated or halogenated as desired. The general formula is: 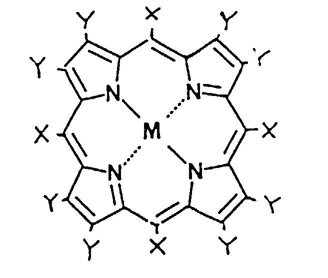 where M is Fe, X is nitro, and Y is nitro, Cl, F, Br or any combination thereof.

Meso-perfluoroalkyl porphyrins can be nitrated in the beta or pyrrolic positions using NO₂ in methylene chloride or nitric acid/sulfuric acid nitrating solutions. The general structure is: 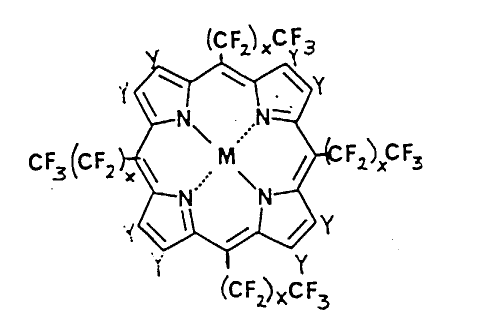 where M is Fe, X is 0-6 and Y is NO₂ and Cl or Br or F.

Cyanometalloporphyrin or cyano/halogenometalloporphyrins can be prepared for example as follows.

Zinc(tetrakispentafluorophenylbeta-octabromoporphine), prepared by the bromination of Zn(tetrakispentafluorophenylporphine) with bromine in carbon tetrachloride, is treated with 9 equivalents of CuCN in pyridine, dimethylformamide, quinoline or mixtures of these solvents at reflux for several hours. After chromatography several of the bromines are replaced with CN groups giving, according to the conditions Zn(TPPF₂₀beta-CN₄₋₈). The zinc is removed by mild treatment with lM HCl and recovered by chromatography on alumina. Fe can be inserted into this H₂(TPPF₂₀betaCN₄₋₈) by treatment with ferrous chloride in DMF leading to Fe(TPPF₂₀beta-CN₄₋₈)Cl.

If the CuCN treatment is conducted under milder conditions some of the bromine groups can be retained leading to mixed bromo/cyano metalloporphyrins. Pyrrolic positions without cyano or bromo substitution can also be brominated, chlorinated or fluorinated leading to complexes of the general structure: 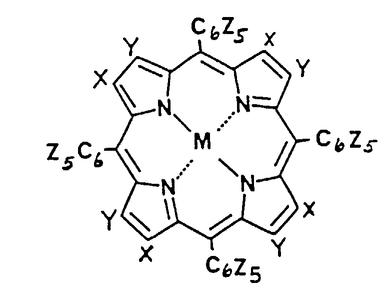 where M is Fe, X is CN, Y is CN, or Cl or Br or F, and Z is H or Cl or F.

Meso- perfluoroalkylporphyrins, as made for example according to the disclosure of EP-A-0471561 , can also be converted to cyano derivatives as shown above.

The general structure is: 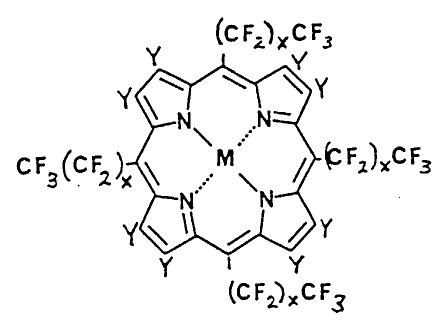 where M is Fe, X is 0-6 and Y is CN and Cl or Br, or F.

In the process of the invention, olefin is contacted with an oxygen-containing gas in the presence of catalyst as disclosed herein. The reaction may be conducted at any suitable temperature, preferably in the range from 25 to 125 degrees C. and at any suitable pressure, preferably in the range from 103.4 to 1034 kPa (15 to 150 psig). The optimum conditions for carrying out a particular reaction within the scope of the invention may be determined by a person skilled in the art in the light of the present specification.

Allylic oxidations which may be performed according to the invention are: (1) propylene to allyl alcohol, acrolein, acrylic acid and byproducts; (2) butylene to crotyl alcohol, crotyraldehyde, crotic acid; (3) isobutylene to methallyl alcohol, methacrolein and methacrylic acid: (4) cyclohexene to 2-cyclohexene-1-ol, 2-cyclohexene-1-one; however any olefin C₃ and higher and any cyclic olefin C₅ or higher can be so oxidized provided it bears an allylic C-H bond and contains no structurally or chemically inhibiting group. The following examples illustrate the invention.

### Example 1

Cyclohexene is oxidized to give predominantly 2-cyclohexene-1-ol and 2-cyclohexene-1-one by contacting oxygen with the olefin at room temperature in the presence of the catalyst, Fe(TPPF₂₀)OH. More than 4000 moles of product were formed per mole of catalyst in forty minutes. This is a rapid rate even on a commercial scale. The reactions started at room temperature but the active iron catalysts promoted rapid and exothermic reactions so that at the end of the forty-minute reaction period the solution temperature had risen to over 70 degrees C. The results are shown in Table I:

**TABLE I**

| RUN | CATALYST | kPa | (PSI) | DEG.C | HRS | OXIDE | ENOL | ENONE | CONV% |
|---|---|---|---|---|---|---|---|---|---|
| 1 | Fe(TPPF₂₀)OH | 689.5 | (100) | 22 | 0.65 | 3.34 | 14.02 | 32.06 | > 17 |
| 2 | Fe(TPPF₂₀)OH | 344.7 | (50) | 23 | 0.65 | 1.75 | 14.17 | 31.13 | > 17 |

In each run, 0.013 mmoles of catalyst were used. Oxygen gas was pressed on a solution of the catalyst in 393 mmoles of cyclohexene. Reaction was allowed to proceed for the designated time (Hrs.) after which the gas was released and the product mixture analyzed by standardized glpc. The temperature (°C) in Runs 1 and 2 is that of the bath when the reaction was started; by the time reaction was terminated the internal temperature had risen to 75 degrees C.; no external heat was applied. "Oxide" in the table is cyclohexene oxide, "Enol" is 2-cyclohexene-1-ol, and "Enone" is 2-cyclohexene-1-one. Conversion ("Conv") is molar conversion of cyclohexene to oxidation products during the reaction time.

### Example 2

2,3-dimethylbutene-2 was oxidized in the same way as in Example 1 using the same catalyst, and an even faster reaction ensued. In order to control the reaction exotherm the 2,3-DMB was diluted with benzene (50%). In the forty minute reaction period the dilute solution had reached over 40 degrees C. and more than 10,000 moles of oxygen had reacted per mole of catalyst used.

### Example 3

Oxidations of cyclohexene were performed with conditions generally similar to those in Example 1, except that the catalyst was dissolved in 293 mmoles of cyclohexene, and the reaction temperature was kept at or below room temperature by immersing the reaction vessel in a room temperature oil bath. The results are shown in Table II. Run 7 is comparative.

**Table II**

| OXIDATIONS OF CYCLOHEXENE CATALYZED BY FERROPORPHYRIN COMPLEXES | | | | | | | | |
|---|---|---|---|---|---|---|---|---|
| RUN | CATALYST | MMOLES | kPa (PSI) | DEG.C | HRS | OXIDE | OL-ONE | CONV |
| 5 | Fe(TPF₂₀Br₈)Cl | 0.013 | 310.2 (45) | 25 | 6 | 0.7 | 31.5 | 11 |
| 6 | Fe(TPPF₂₀Br₈)Cl | 0.013 | 310.2 (45) | 25 | 6 | 0.5 | 22.4 | 8 |
| 7 | Fe(TPP)Cl | 0.013 | 310.2 (45) | 25 | 6 | tr | tr | 0 |

In obtaining the data of Table II, oxygen gas was pressed on a solution of the catalyst in 293 mmoles of cyclohexene. Reaction was allowed to proceed for the designated time (Hrs) after which the gas was released and and the product mixture analyzed by standardized glpc. "Oxide" in the table is the percent of cyclohexene oxide in the reaction product. "Ol-one" is the percent of 2-cyclohexene-l-ol plus 2-cyclohexene-1-one in the reaction product. Conversion ("Conv") is molar conversion of cyclohexene to oxidation products during the reaction time.

Table II shows that the catalysts used according to the invention in Runs 5 and 6 were effective in allylic oxidation of cyclohexene, whereas the catalyst used in Run 7 was not. Furthermore, when oxidations of cyclohexene were attempted by the procedure of Example 3, but using the following catalysts: ferrous acetylacetonate; ferric acetylacetonate, no reaction was obtained in a three hour period.

## Claims

1. Process for allylic oxidation of olefins which comprises contacting an olefin with an oxygen-containing gas, in the absence of a single oxygen atom donor, and in the presence of a catalyst having the formula: 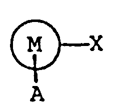 where M is Fe, 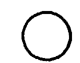 is a porphyrin ligand, X is an electron-withdrawing substituent for hydrogen in the ligand wherein the electron-withdrawing substituent is halogen, nitro or cyano, such that when X is halogen the degree of substitution is at least 70% and when X is nitro or cyano the degree of substitution is at least 10%, and A is an anion or is absent, to effect allylic oxidation of the olefin.

2. Process according to claim 1 wherein X is halogen.

3. Process according to claim 2 wherein X is fluoride.

4. Process according to claim 2 wherein the ligand is perhalogenated.

5. Process according to claim 2 wherein the catalyst is tetrakis(pentafluorophenyl)porphyrinato iron(III)chloride, Fe(TPPF₂₀)Cl.

6. Process according to claim 4 wherein the catalyst is tetrakis(pentafluorophenyl)β-octabromoporphyrinato iron(III)chloride, Fe(TPPF₂₀Br₈)Cl.

7. Process according to claim 1 wherein the catalyst is tetrakis(pentafluorophenyl)β-octachloroporphyrinato iron(III)chloride, Fe(TPPF₂₀Cl₈)Cl.

8. Process according to claim 1 wherein the catalyst is tetrakis(pentafluorophenyl)porphyrinatoiron(III)hydroxide, Fe(TPPF₂₀)OH.

9. Process according to claim 1 wherein X is nitro.

10. Process according to claim 1 wherein X is cyano.

## Patentansprüche

1. Verfahren zur allylischen Oxidation von Olefinen, bei dem ein Olefin in Abwesenheit eines Donors für ein einzelnes Sauerstoffatom und in Gegenwart eines Katalysators der Formel 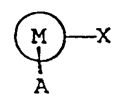 mit einem Sauerstoff enthaltenden Gas in Kontakt gebracht wird,
worin M für Fe steht, 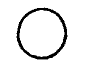 ein Porphyrinligand ist, X ein Elektronen anziehender Substituent für Wasserstoff in dem Liganden ist, wobei der Elektronen anziehende Substituent ein Halogenatom, eine Nitro- oder Cyangruppe ist, unter der Bedingung, daß dann, wenn X Halogen ist, der Substitutionsgrad mindestens 70 % ist und wenn X eine Nitro- oder Cyangruppe ist, der Substitutionsgrad mindestens 10 % beträgt, und A ein Anion darstellt oder nicht vorhanden ist, um die allylische Oxidation des Olefins durchzuführen.

2. Verfahren nach Anspruch 1, wobei X Halogen bedeutet.

3. Verfahren nach Anspruch 2, wobei X Fluorid ist.

4. Verfahren nach Anspruch 2, wobei der Ligand perhalogeniert ist.

5. Verfahren nach Anspruch 2, wobei der Katalysator Tetrakis(pentafluorphenyl)porphyrinato-eisen(III)chlorid, Fe(TPPF₂₀)Cl, ist.

6. Verfahren nach Anspruch 4, wobei der Katalysator Tetrakis(pentafluorphenyl)β-octabromporphyrinato-eisen(III)chlorid, Fe(TPPF₂₀Br₈)Cl, ist.

7. Verfahren nach Anspruch 1, wobei der Katalysator Tetrakis(pentafluorphenyl)β-octachlorporphyrinatoeisen(III)chlorid, Fe(TPPF₂₀Cl₈)Cl, ist.

8. Verfahren nach Anspruch 1, wobei der Katalysator Tetrakis(pentafluorphenyl)porphyrinato-eisen(III)hydroxid, Fe(TPPF₂₀)OH, ist.

9. Verfahren nach Anspruch 1, wobei X eine Nitrogruppe ist.

10. Verfahren nach Anspruch 1, wobei X eine Cyangruppe ist.

## Revendications

1. Procédé d'oxydation allylique d'oléfines, caractérisé en ce que l'on met une oléfine en contact avec un gaz contenant de l'oxygène, en l'absence d'un donneur d'atome d'oxygène unique et en présence d'un catalyseur répondant à la formule : 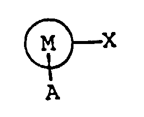 dans laquelle M est Fe, 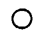 est un ligand du type porphyrine, X représente un substituant de l'hydrogène, soutirant des électrons, dans le ligand, où le substituant soutirant des électrons est un atome d'halogène, un radical nitro ou cyano, en une manière telle que lorsque X représente un atome d'halogène, le degré de substitution soit d'au moins 70% et que lorsque X représente un radical nitro ou cyano, le degré de substitution soit d'au moins 10% et A représente un anion, ou bien est absent, de manière à réaliser l'oxydation allylique de l'oléfine.

2. Procédé suivant la revendication 1, caractérisé en ce que X représente un atome d'halogène.

3. Procédé suivant la revendication 2, caractérisé en ce que X représente un atome de fluor.

4. Procédé suivant la revendication 2, caractérisé en ce que le ligand est perhalogéné.

5. Procédé suivant la revendication 2, caractérisé en ce que le catalyseur est le chlorure de tétrakis(pentafluorophényl)porphyrinatofer(III), Fe(TPF₂₀)Cl.

6. Procédé suivant la revendication 4, caractérisé en ce que le catalyseur est le chlorure de tétrakis(pentafluorophényl)β-octabromoporphyrinatofer(III), Fe(TPPF₂₀Br₈)Cl.

7. Procédé suivant la revendication 1, caractérisé en ce que le catalyseur est le chlorure de tétrakis(pentafluorophényl)β-octachloroporphyrinatofer(III), FE(TPPF₂₀Cl₈)Cl.

8. Procédé suivant la revendication 1, caractérisé en ce que le catalyseur est l'hydroxyde de tétrakis(pentafluorophényl)porphyrinatofer(III), Fe(TPPF₂₀)OH.

9. Procédé suivant la revendication 1, caractérisé en ce que X représente le radical nitro.

10. Procédé suivant la revendication 1, caractérisé en ce que X représente le radical cyano.
